Europäisches Patentamt

European Patent Office

Office européen des brevets

(11) Publication number: **0 249 398**
**A2**

## (12) EUROPEAN PATENT APPLICATION

(21) Application number: **87304946.4**

(22) Date of filing: **04.06.87**

(51) Int. Cl.4: **A61K 7/16 , A61K 7/18**

(30) Priority: **09.06.86 US 872356**
**12.09.86 US 907138**
**13.05.87 US 47374**

(43) Date of publication of application:
**16.12.87 Bulletin 87/51**

(84) Designated Contracting States:
**AT BE CH DE ES FR GB GR IT LI LU NL SE**

(71) Applicant: **THE PROCTER & GAMBLE COMPANY**
**One Procter & Gamble Plaza**
**Cincinnati Ohio 45202(US)**

(72) Inventor: **Seus, John David**
**3762 Ault Park Avenue**
**Cincinnati Ohio 45208(US)**

(74) Representative: **Brooks, Maxim Courtney et al**
**Procter & Gamble (NTC) Limited Whitley Road Longbenton**
**Newcastle-upon-Tyne NE12 9TS(GB)**

(54) **Oral compositions.**

(57) Compositions which provide enhanced anticalculus efficacy.

## ORAL COMPOSITIONS

## CROSS REFERENCE TO RELATED APPLICATIONS

This is a continuation-in-part of my copending application having Serial No. 907,138, filed September 12, 1986, which is a continuation-in-part of application having Serial No. 872,356, filed June 9, 1986, now abandoned.

## TECHNICAL FIELD

The present invention relates to oral compositions which provide anticalculus efficacy.

## BACKGROUND OF THE INVENTION

Dental calculus, or tartar as it is sometimes called, is a deposit which forms on the surface of the teeth in humans and lower animals at the gingival margin. Supragingival calculus appears principally in the areas near the orifices of the salivary ducts; e.g., on the lingual surfaces of the lower anterior teeth and on the buccal surfaces of the upper first and second molars, and on the distal surfaces of the posterior molars.

Mature calculus consists of an inorganic portion which is largely calcium phosphate arranged in a hydroxyapatite crystal lattice structure similar to bone, enamel and dentine. An organic portion is also present and consists of desquamated epithelial cells, leukocytes, salivary sediment, food debris and various types of microorganisms.

As the mature calculus develops, it becomes visibly white or yellowish in color unless stained or discolored by some extraneous agent. In addition to being unsightly and undesirable from an aesthetic standpoint, the mature calculus deposits are regarded by many as a constant source of mechanical irritation of the gingiva.

A wide variety of chemical and biological agents have been suggested in the art to retard calculus formation or to remove calculus after it is formed. Mechanical removal of this material periodically by the dentist is, of course, routine dental office procedure.

The chemical approach to calculus inhibition generally involves chelation of calcium ion and/or crystal growth inhibition in the mouth which prevents the calculus from forming and/or breaks down mature calculus by removing calcium.

An exemplary approach to calculus reduction involves the use of soluble pyrophosphate salts as disclosed in U.S. Patent 4,515,772, issued May 7, 1985 to Parran et al, incorporated herein by reference.

The compositions taught by Parran et al. comprise from about 0 to about 70% of a dental abrasive; an amount of a fluoride source sufficient to supply from about 50 ppm to about 3,500 ppm of fluoride ions to reduce, for example, the hydrolysis of the pyrophosphate salt to ortho phosphates by the action of pyrophosphatase; an amount of soluble pyrophosphate salt sufficient to provide at least about 1.5% $(P_2O_7)^{-4}$; and from about 2% to about 95% water; and have a pH of from about 6 to about 10.

The compositions of Parran, et al. provide exemplary anticalculus performance. Additionally, the compositions have good aesthetics (e.g. little grittiness) and reduce the possibility of pyrophosphate crystals being formed. In compositions containing relatively high levels of sodium ions through the use of tetrasodium pyrophosphate as in U.S. Patent 3,927,202, December 16, 1975 to Harvey, crystals can form. Parran et al. reduces crystal formation by the use of dialkalimetal pyrophosphate salts, at least 0.50%, in their compositions.

It has now been surprisingly found that the formation of pyrophosphate crystals can be reduced in yet another way. If the compositions have potassium ions added so that the ratio of sodium ions to potassium ions is appropriate, crystal formation is reduced without resort to high levels dialkali metal pyrophosphate salts. The sources of the sodium and potassium ions can be any reasonable source such as the pyrophosphate salts, sweeteners such as cyclamate or saccharin or surfactants such as alkyl sulfates.

It is believed that pyrophosphate crystals which are avoided in the present invention have the empirical formula (neglecting the presence of any included water) of $Na_xK_yH_z(P_2O_7)$ wherein x and y individually are numbers having values of from 0 to about 4, z is a number a value of from 0 to 3 and x + x + z = 4. If crystals are not prevented from growing as by the Parran et al. invention or the present invention they can reach lengths of from about 2mm to about 8mm after a period as short as 8 weeks at 10°C.

It is an object of the present invention to provide oral compositions which deliver an improved anticalculus benefit, which do not suffer from pyrophosphate crystal formation and are aesthetically pleasing (e.g. little grittiness and no large consumer noticeable crystals).

It is a further object of the present invention to provide a process for making such oral compositions.

It is a still further object of the present invention to provide anticalculus methods using soluble pyrophosphate salts.

## SUMMARY OF THE INVENTION

It has been surprisingly discovered that the growth of crystals of pyrophosphate salts in oral compositions, can be reduced if the ratio of sodium ions to potassium ions is controlled.

The present invention provides oral compositions based on soluble pyrophosphate sources and having an anticalculus benefit, which oral compositions are physically stable and aesthetically pleasing. The compositions comprise:

(a) from about 0% to about 70% by weight of a dental abrasive compatible with pyrophosphate ions and fluoride ions;

(b) an amount of a fluoride ion source sufficient to supply from about 50 ppm to about 3,500 ppm of fluoride ions;

(c) an amount of at least one pyrophosphate source sufficient to provide at least about 1.5% $(P_2O_7)^{-4}$; and

(d) from about 2% to about 95% water. wherein the pH of said composition is from about 6.0 to about 10.0, said composition contains sodium ions and potassium ions in a ratio of sodium ions to potassium ions which reduces the formation of crystals to pyrophosphate salts and said composition contains not more than about 0.5% total dialkali metal pyrophosphate sources.

The present invention also embraces a process for preparing oral compositions comprising:

(a) from 0% to about 70% by weight of a dental abrasive compatible with pyrophosphate ions and fluoride ions;

(b) an amount of a fluoride ion source sufficient to supply from about 50 ppm to about 3,500 ppm of fluoride ions;

(c) at least about 0.5% of at least one cyclamate salt;

(d) an amount of a pyrophosphate source sufficient to provide at least 1.5% $(P_2O_7)^{-4}$; and

(e) from about 2% to about 95% water; wherein the pH of said composition is from about 6.0 to about 10.0 and said composition contains sodium ions and potassium ions in a ratio of sodium ions to potassium ions which reduces the formation of crystals of pyrophosphate salts and said process comprising the mixing of the said aforementioned components in such a manner that all of said cyclamate salts are incorporated into said composition prior to the incorporation therein of said pyrophosphate salts.

The present invention includes a method for reducing the incidence of calculus on dental enamel which process comprises contacting the enamel surfaces in the mouth with an oral composition comprising a soluble pyrophosphate source capable of providing to the composition at least about 1.5% $(P_2O_7)^{-4}$, from about 50 ppm to about 3,500 ppm fluoride ions, not more than about 0.5% total dialkali metal salt, and sodium and potassium ions wherein the ratio of the sodium ions to the potassium ions is from about 0.2:1 to about 5.7:1, preferably from about 0.4:1 to about 2.6:1.

The present invention also includes a method for reducing the incidence of calculus on dental enamel which process comprises contacting the enamel surfaces in the mouth with an oral composition comprising a soluble pyrophosphate source capable of providing to the composition at least about 1.5% $(P_2O_7)^{-4}$, from about 50 ppm to about 3,500 ppm fluoride ions, at least about 0.4% cyclamate ions, and sodium ions and potassium ions wherein the ratio of the sodium ions to the potassium ions is from about 0.2:1 to about 5.7:1.

Unless otherwise specified, all percentages herein are by weight of the total composition, and all ratios are weight ratios of the materials involved. The abbreviation "ppm" refers to parts (by weight) of the noted component per one million ($10^6$) parts of total composition.

By the term "comprising", as used herein, is meant that various additional components can be conjointly employed in the compositions of this invention as long as the listed materials perform their intended functions.

By the term "carrier", as used herein, is meant a suitable vehicle which is pharmaceutically acceptable and can be used to apply the present compositions in the oral cavity.

## DETAILED DESCRIPTION OF THE INVENTION

The oral compositions contemplated by the present invention include mouthwashes, liquid dentifrices, and toothpastes. All three of these embodiments are well known in the art.

The essential as well as optional components of the compositions of the present invention are described in the following paragraphs.

### Dental Abrasive

The abrasives useful in the oral composition of the present invention include many different materials. The material selected must be one which has good compatibility with both fluoride ions and pyrophosphate ions. In this respect the abrasive must be able to allow for the quantities of fluoride ions and pyrophosphate ions indicated herein below. Suitable abrasives include betaphase calcium pyrophosphate prepared in accordance with the teaching of Schweizer, U.S. Patent 3,112,247, November 26, 1963. The beta-phase calcium pyrophosphate is prepared by heating gamma-phase calcium pyrophosphate to 700-900°C to change at least 50% of the gamma-phase to beta-phase and then immediately cooling. Another class of abrasives for use herein is the particulate thermosetting polymerized resins as described by Cooley et al in U.S. Patent 3, 070,510, December 25, 1962. Suitable resins include, for example, melamines, phenolics, ureas, melamineureas, melamineformaldehydes, urea-formaldehydes, melamine-urea-formaldehydes, cross-linked epoxides, and cross-linked polyesters.

Silica dental abrasives are also useful in the present compositions. The silica abrasive polishing material generally has an average particle size ranging between about 0.1 to 30 microns, preferably between 5 and 15 microns. The abrasive can be precipitated silica or silica gels such as the silica xerogels described in Pader et al, U.S. Patent 3,538,230, March 2, 1970 and DiGiulio, U.S. Patent 3,862,307, January 21, 1975, incorporated herein by reference. Preferred are the silica xerogels marketed under the tradename "Syloid" by the W. R. Grace & Company, Davison Chemical Division. Preferred precipitated silica materials are those marketed by the J. M. Huber Corporation under the tradename, "Zeodent", particularly the silica carrying the designation Zeodent 119. These silica abrasives are described in U.S. Patent 4,340,583, July 29, 1982, incorporated herein by reference.

Other suitable abrasives include alumina, and the insoluble metaphosphates such as insoluble sodium metaphosphate (IMP). Mixtures of abrasives can also be used. In any case, the total amount of abrasive in the dentifrice (toothpaste) embodiments of this invention can range from 10% to 70% by weight of the dentifrice. Preferably, toothpastes contain from 10% to 50% by weight of abrasive. (Naturally, when the composition is a mouthwash the level of abrasive can be as low as 0%)

The preferred abrasives are the ß-phase calcium pyrophosphate of U.S. Patent 3,112,247; alumina; insoluble metaphosphate; the resinous abrasives of U.S. Patent 3,070,510; and the silica abrasives since they are more compatible with the agents. Most preferred are the silica abrasives.

### Fluoride Ion Source

The second essential component of the oral compositions herein is a fluoride ion source. The number of such sources is great and includes those disclosed in U.S. Patent 3,535,421, October 20, 1970 to Briner et al, incorporated herein by reference. Typical materials include:

Stannous fluoride, potassium fluoride, lithium fluoride, cesium fluoride, ammonium fluoride, sodium fluoride, cupric fluoride, indium fluoride, stannous fluorozirconate, lead fluoride, ferric fluoride, nickel fluoride, paladium fluoride, silver fluoride, zinc fluoride, zirconium fluoride, hexylamine hydrofluoride, laurylamine hydrofluoride, myristylamine hydrofluoride, decanolamine hydrofluoride, octadecenylamine hydrofluoride, myristoxy amine hydrofluoride, diethylaminoethyloctoylamide hydrofluoride, diethanolamineoethyloleylamide hydrofluoride, diethanolaminopropyl-N'-octadecenylamine dihydrofluoride, 1-ethanol-2-hexadecylimidazoline

4

dihydrofluoride, octoylethanolamine hydrofluoride, octyltrimethylammonium fluoride, dodecylethyldimethylammonium fluoride, tetraethylammonium fluoride, dilauryldimethylammonium fluoride. $\Delta$-8,9-octadecenylbenzyldimethylammonium fluoride, dioctyldiethylammonium fluoride, cyclohexylcetyldimethylammonium fluoride, furfuryllauryldimethylammonium fluoride, phenoxyethylcetyldimethylammonium fluoride, N:N'-tetramethyl-N:N;-dilaurylethylenediammonium difluoride, N-cetylpyridinium fluoride, N:N-dilauryl-morpholinium fluoride, N-myristyl-N-ethylmorpholinium fluoride, N-(octylaminocarbonylethyl)-N-benzyldimethylammonium fluoride, N($\beta$-hydroxydodecyl)trimethylammonium fluoride, N-phenyl-N-hexadecyldiethylammonium fluoride, N-cyclohexyl-N-octadecyldimethylammonium fluoride, N-(2-carbomethoxyethyl)-N-benzyldimethylammonium fluoride, N-(2-carbocyclohexoxyethyl)-N-myristyldimethylammonium fluoride, N-(2carbobenzyloxyethyl)-N-dodecyldimethylammonium fluoride, N-[2-(N:N'-dimethylaminocarbonyl)-ethyl]-N-dodecyldiethylammonium fluoride, N-carboxymethyl-N-cicosyldimelthylammonium fluoride, betaine hydrofluoride, sarcosine stannous fluoride, alanine stannous fluoride, glycine potassium fluoride, sarcosine potassium fluoride, glycine hydrofluoride, lysine hydrofluoride, alanine hydrofluoride, betaine zirconium fluoride, sodium monofluoro phosphate and mixtures thereof. Sodium fluoride is the preferred fluoride source.

The amount of the fluoride ion source should be sufficient to provide from about 50 ppm to 3500 ppm, preferably from about 500 ppm to 3000 ppm of fluoride ions.

Pyrophosphate Salts

The pyrophosphate sources useful in the present compositions include, for example, dialkali metal pyrophosphate salts, tetraalkali metal pyrophosphate salts, and mixtures thereof. $Na_2H_2P_2O_7$, $K_2H_2P_2O_7$, $Na_4P_2O_7$ and $K_4P_2O_7$ in their unhydrated as well as hydrated forms are the preferred species. The levels of each of these species which preferably are used in the compositions are as follows (all are in the unhydrated form).

$Na_2H_2P_2O_7$: 0 - 0.5%

$K_2H_2P_2O_7$: 0 - 0.5%

$Na_4P_2O_7$: 0 - 8.0%

$K_2P_2O_7$: 0 - 8.0%

The minimum amount of pyrophosphate ions, $(P_2O_7)^{-4}$, required to be provided to the present compositions is 1.5%, preferably 2.0%, and can be provided, for example by mixtures of tetrasodium and tetrapotassium sources or mixtures which include the dialkali metal source. It is to be appreciated that other pyrophosphate forms (e.g. $HPO_4^{-3}$) may be present when the salt is totally dissolved and a pH established.

The pyrophosphate salts are described in more detail in Kirk & Othmer, Encyclopedia of Chemical Technology, Second Edition, Volume 15, Interscience Publishers (1968) incorporated herein by reference.

Water

Water is another essential component of the oral compositions of this invention. Water employed in the preparation of commercially suitable oral compositions should preferably be of low ion content and free of organic impurities. Water generally comprises from about 2% to about 95%, preferably from about 20% to about 95% of the oral compositions useful in this invention.

Additional Components

In addition to the above described essential components, the compositions of this invention can contain a variety of optional, conventional oral composition components. Such optional ingredients include sudsing agents, binders, flavoring agents, humectants, sweetening agents, antiplaque agents, coloring agents, and pigments.

A preferred optional ingredient is a sudsing agent. Suitable sudsing agents are those which are reasonably stable and form suds throughout a wide pH range, i.e., nonsoap anionic, non-ionic, cationic, zwitterionic and amphoteric organic synthetic detergents. Sudsing agents of these types are described more fully in Agricola et al, U.S. Patent 3,959,458, May 25, 1976, and in Haefele, U.S. Patent 3,937,807, February 10, 1976. Both of these patents are incorporated herein by reference.

Anionic sudsing agents useful herein include the water-soluble salts of alkyl sulfates having from 10 to 18 carbon atoms in the alkyl radical and the water-soluble salts of sulfonated monoglycerides of fatty acids having from 10 to 18 carbon atoms. Sodium lauryl sulfate and sodium coconut monoglyceride sulfonates are examples of anionic surfactants of this type. Mixtures of anionic surfactants can also be employed.

The nonionic sudsing agents which can be used in the compositions of the present invention can be broadly defined as compounds produced by the condensation of alkylene oxide groups (hydrophilic in nature) with an organic hydrophobic compound which may be aliphatic or alkylaromatic in nature. Examples of suitable nonionic sudsing agents include the Pluronics, polyethylene oxide condensates of alkyl phenols, products derived from the condensation of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

The zwitterionic synthetic sudsing agents useful in the compositions of the present invention can be broadly described as derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate, orthophosphonate.

The cationic sudsing agents useful in the compositions of the present invention can be broadly defined as quaternary ammonium ompounds having one long alkyl chain containing from about 8 to about 18 carbon atoms such as lauryl trimethylammonium chloride; cetyl pyridinium chloride; cetyl trimethylammonium bromide; di-isobutylphenoxyethoxyethyl-dimethylbenzylammonium chloride; coconutalkyl-trimethylammonium nitrate; cetyl pyridinium fluoride; etc.

The amphoteric sudsing agents useful in the present invention can be broadly described as derivatives of aliphatic secondary and tertiary amines in which the aliphatic radical can be straight chain or branched and wherein one of the aliphatic substituents contains from about 8 to about 18 carbon atoms and one contains an anionic water-solubilizing group, e.g., carboxylate, sulfonate, sulfate, phosphate, or phosphonate.

The sudsing agent can be present in the compositions of this invention in an amount from about 0% to about 10% by weight of the total composition.

Conventional mouthrinse composition components can comprise the carrier for the mouthrinse embodiment of the present invention. Mouthrinses generally comprise from about 20:1 to about 2:1 of a water/ethyl alcohol solution and preferably other ingredients such as flavors, humectants, and sudsing agents such as those mentioned above for dentifrices. The humectants, such as glycerin and sorbitol give a moist feel to the mouth. Generally, on a weight basis the mouthrinses of the invention comprise 5% to 60% (preferably 1% to 25%) ethyl alcohol, 0% to 20% (preferably 5% to 20%) of a humectant, 0% to 2% (preferably 0.01% to 0.20%) emulsifying agents, 0% to 0.3% (preferably 0.03% to 0.3%) flavoring agent, and the balance water.

Flavoring agents can also be added to the instant compositions. Suitable flavoring agents include oil of wintergreen, oil of peppermint, oil of spearmint, oil of sassafras, and oil of clove. These agents are generally used at levels up to about 2%.

Binders can also be used with the toothpastes of the present inventions. Such binders include, for example, xanthan gum, carrageenan (Irish moss, Viscarin TP-5 which is a iota carrageenan), carboxy celluloses, and carboxyvinyl polymers. These binders are generally present at a level of from about 0.1% to 2%.

Bis-biguanide antiplaque agents can also optionally be added to the compositions of this invention. Such agents include chlorhexidine (1,6-bis{N⁵-p-chlorophenyl-N¹-biguanido]hexane), the soluble and insoluble salts thereof and related materials such as 1,2-is(N⁵-p-trifluoromethylphenyl-N¹-biguanido)ethane and are described more fully in Haefele, U.S. Patent 3,923,002, January 20, 1976, Haefele, U.S. Patent 3,937,807, February 10, 1976, Procter & Gamble, Belgian Patent 843,244,, published December 22, 1976, and Proctor & Gamble, Belgian Patent 844,764, published January 31, 1977. These patents are incorporated herein by reference.

If present, the optional antiplaque agents generally comprise from about 0% to about 5% by weight of the compositions herein.

Another optional component of the compositions herein is a humectant as noted above. The humectant serves to keep the toothpaste compositions from hardening upon exposure to air and in mouthrinses give a moist feel to the mouth. Certain humectants can also impart desirable sweetness of flavor to mouthrinse and toothpaste compositions. The humectant, on a pure humectant basis, generally comprises from about 0% to 70%, preferably from about 0% to 55%, by weight of the compositions herein.

Suitable humectants for use in this invention include edible polyhydric alcohols such as glycerine, sorbitol, xylitol and propylene glycol. Sorbitol is frequently employed as a 70% aqueous solution known as Sorbo[R].

Sweetening agents can also be used in the compositions of this invention. Materials such as saccharin, dextrose, levulose, thaumatin, aspartame, D-tryptophan, dihydrochalcones, and acesulfame are suitable. Sodium cyclamate is a preferred sweetening agent. Sweetening agents are generally used at levels of from about 0.1% to about 5% by weight.

The physical instability problem noted above, the growth of pyrophosphate crystals, is observed in oral compositions when the level of sodium ions is sufficiently great to cause the pyrophosphate ions to form an insoluble complex. It has been surprisingly discovered that the growth of the pyrophosphate crystals can be significantly inhibited if the ratio of sodium ions to potassium ions in the oral composition is controlled. The ratio of sodium ions to potassium ions is maintained in the range of from about 0.2:1 to about 5.7:1, more preferably from about 0.4:1 to about 2.6:1. The source of the sodium and potassium ions in the oral composition is immaterial. As a practical matter, the ratio of sodium and potassium ions is preferably controlled by adjusting the relative levels of the soluble salts, such as the soluble pyrophosphates, used in the oral compositions.

The pH of the oral compositions herein is in the range of 6.0 to 10.0, preferably from 7.0 to 9.0. The pH is preferably achieved through a proper balancing of the pyrophosphate sources or by the addition of an alkaline or acidic agent.

The compositions of the present invention can be prepared by any method conventional in the art. When cyclamate salts are used as the sweetening agents, it has been observed that crystals of potassium cyclamate can form. The size of these crystals can be controlled at an acceptable level (i.e. less than about 1000 microns) if the cyclamate salts are incorporated into the system prior to the incorporation therein of the pyrophosphate sources.

The compositions of the present invention are used in conventional ways, such as in brushing the teeth or rinsing the mouth, to reduce the incidence of dental calculus. That is to say, the enamel surfaces in the mouth are contacted by the compositions hereinbefore described.

The following examples are provided by way of illustration and not limitation.

EXAMPLE 1

A toothpaste having the following composition is prepared. The components are added to a mixer in such a manner that the sodium cyclamate is introduced into the system prior to the introduction therein of the tetrapotassium pyrophosphate solution and the sodium acid pyrophosphate.

| | |
|---|---|
| Tetrapotassium Pyrophosphate Solution (65%) | 6.80% (weight) |
| Sodium Acid Pyrophosphate | 0.40 |
| Flavor | 1.04 |
| PEG-6 | 2.00 |
| Glycerin | 8.00 |
| Sodium Fluoride | 0.24 |
| Sorbitol Solution (70%) | 32.00 |
| Silica Abrasive | 20.00 |
| Distilled Water | 21.02 |
| Sodium Alkyl Sulfate Solution (27.9%) | 4.00 |

| | |
|---|---|
| Sodium Cyclamate | 3.27 |
| Rutile Titanium Dioxide | 0.53 |
| Xanthan Gum | 0.40 |
| Carbopol 940 | 0.25 |
| FD&C Blue No. 1 Solution (1%) | 0.05 |
| | 100.00 |

The ratio of sodium to potassium in this toothpaste is 0.32, the pH is adjusted to 7.6 and the paste contains 1100 ppm fluoride ions. The toothpaste is stable (i.e. crystals of pyrophosphate do not form) on storage at 10°C for more than eight weeks and the paste provides anticalculus efficacy.

EXAMPLE 2

This tcothpaste is prepared as in Example 1, except the following composition is used:

| | |
|---|---|
| Tetra potassium pyrophosphate solution (65%) | 4.50% (weight) |
| Sodium Acid Pyrophosphate | 0.30 |
| Flavor | 1.04 |
| PEG-6 | 1.00 |
| Glycerin | 8.00 |
| Sodium Fluoride | 0.24 |
| Sorbitol Solution (70%) | 32.00 |
| Silica Abrasive | 20.00 |
| Distilled Water | 22.22 |
| Sodium Alkyl Sulfate Solution (27.9%) | 4.00 |
| Sodium Cyclamate | 3.27 |
| Rutile Titanium Dioxide | 0.53 |
| Xanthan Gum | 0.60 |
| Carbopol 940 | 0.25 |
| FD&C Blue No. 1 Solution (1%) | 0.05 |
| | 100.00 |

The ratio of sodium to potassium ions in this toothpaste is 0.47, the pH is adjusted to 7.6 and the paste contains 1100 ppm fluoride ions.

This toothpaste is also stable for more than eight weeks at 10°C and provides anticalculus activity.

EXAMPLE 3

The following composition is also exemplary of the present invention.

| | |
|---|---|
| Distilled Water | 24.432% (weight) |
| Sorbitol Solution (70% Solution) | 32.000 |
| Tetrasodium Pyrophosphate | 4.480 |
| Tetrapotassium Pyrophosphate (65% Solution) | 1.040 |
| Sodium Saccharin | 0.286 |
| Sodium Fluoride | 0.243 |
| Silica Abrasive | 22.000 |
| Flavor | 1.044 |
| FD&C Blue #1 Solution (1% Solution) | 0.050 |
| Sodium Alkyl Sulfate Solution (27.9% Solution) | 4.000 |
| Carbopol 940 | 0.350 |
| Iota Carrageenan | 0.550 |
| Glycerin | 8.000 |
| $T_1O_2$ | 0.525 |
| PEG-6 | 1.000 |

The ratio of sodium to potassium ions in this toothpaste is 5.66, the pH is adjusted to 8.2 and the paste contains 1100 ppm fluoride ions.

The amounts of sodium and potassium ions in the above composition are determined by simply multiplying the fraction of either ion in a particular component by the weight of that component and totalling the individual amounts.

When the above composition is tested for anticalculus activity, it is found to be effective in an in-vitro model.


Claims

1. An oral composition comprising:
(a) from about 0% to about 70% by weight of a dental abrasive compatible with pyrophosphate ions and fluoride ions;
(b) an amount of fluoride ion source sufficient to supply from about 50 ppm to about 3,500 ppm of fluoride ions;
(c) an amount of at least one pyrophosphate source sufficient to provide at least 1.5% $((P_2O_7)^{-4}$; and
(d) from about 2% to about 95% water;
wherein the pH of said composition is from about 6.0 to about 10.0, said composition contains sodium ions and potassium ions in a ratio of sodium ions to potassium ions of from about 0.2:1 to about 5.7:1 and said compositions contain not more than about 0.5% total dialkali metal pyrophosphate sources.

2. The composition of Claim 1 wherein said ratio of said sodium ions to said potassium ions is from about 0.4:1 to about 2.6:1.

3. The composition of Claim 2 in the form of a dentifrice wherein the pyrophosphate source is selected from the group consisting of dialkali metal pyrophosphate salts, tetraalkali metal pyrophosphate salts and mixtures thereof.

4. The composition of Claim 3 wherein the pyrophosphate source is a mixture of tetrasodium pyrophosphate and tetrapotassium pyrophosphate salts.

5. The composition of Claim 4 wherein said dental abrasive is selected from the group consisting of beta-phase calcium pyrophosphate, insoluble metaphosphates, alumina, thermosetting polymerized resins, silica, and mixtures thereof.

6. The composition of Claim 5 wherein said composition comprises at least about 0.5% sodium cyclamate.